# EUROPEAN PATENT SPECIFICATION

(11) **EP 1 051 781 B1**
(45) Date of publication and mention of the grant of the patent: **23.03.2005**
(21) Application number: 99905449.7
(22) Date of filing: 20.01.1999
(51) Int. Cl.: H01S 3/08, A61F 9/00, H01S 3/00, B23K 26/06, G02B 27/09

(54) **LASER DELIVERY SYSTEM WITH DIFFRACTIVE OPTIC BEAM INTEGRATION**
LASERLICHTQUELLE MIT DIFFRAKTIVER OPTISCHER STRAHLINTEGRATION
SYSTEME A DECHARGE PAR LASER AVEC INTEGRATION DE FAISCEAUX OPTIQUES A DIFFRACTION

(30) Priority: 29.01.1998 US 15841
(43) Date of publication of application: 15.11.2000
(73) Proprietor: VISX INCORPORATED, Santa Clara, CA 95051-0703 (US)
(72) Inventor: CAUDLE, George, San Jose, CA 95118 (US); LEMBERG, Vladimir, Belmont, CA 94002 (US)
(74) Representative: Milhench, Howard Leslie
(86) International application number: PCT/US1999/001281
(87) International publication number: WO 1999/039410

(56) References cited:
- DE-A- 4 337 842
- DE-C- 4 441 579
- DE-C- 19 619 481
- US-A- 5 376 086
- US-A- 5 717 518

## Description

### FIELD OF THE INVENTION

This invention relates generally to light beam systems for modifying the spatial intensity distribution of a beam, and more particularly to a light beam system for modifying the spatial intensity distribution of an excimer laser beam to produce a beam of substantially uniform intensity for tissue ablation.

### BACKGROUND OF THE INVENTION

Excimer lasers have been used for various applications, including tissue ablation such as corneal ablation and other surgical procedures. The cross-section of the intensity profile of a typical excimer laser beam is typically not spatially uniform. In general, the beam has a generally rectangular cross-section. The intensity along the long axis of the rectangular beam is substantially constant over the central portion of the beam. The intensity along the short axis of the beam is substantially gaussian. Therefore, the divergence of the excimer laser beam is different along the two axes. As a result, the beam changes shape as it is emitted and travels away from the excimer laser.

Producing a laser beam with a substantially uniform intensity is important in many surgical procedures such as tissue ablation, particularly in corneal ablation for refractive correction or therapeutic purposes. In addition, the laser beam should maintain the shape required by the ablation algorithm throughout the ablation procedure.

Various methods have been used to modify the spatial exposure or intensity distribution of laser beams. To generate a beam with more uniform intensity over the beam cross-section at the plane in which the ablation takes place, researchers have modified the laser discharge volume and the resonator optics to increase uniformity and reduce divergence in the beam. An aperture in a mask selects a nearly uniform portion of the beam by truncating the remaining portion of the beam. The aperture is imaged about an ablation plane such as the corneal plane. Alternatively, if the beam divergence is sufficiently low, the beam selected by the aperture may be projected directly to the corneal plane.

Another method of improving beam intensity employs complex optical systems such as a set of mirrors, prisms, or lenses to break the beam into a series of beamlets. The beamlets are overlapped in a manner to produce a uniform intensity through an aperture of a mask. The aperture is imaged onto the corneal plane.

Still another method employs a rotatable mask formed with one or more apertures having a geometric spiral shape to modify the spatial intensity distribution of a beam, as disclosed in U.S. Patent No. 5,651,784 issued to Klopotek for "ROTATABLE APERTURE APPARATUS AND METHODS FOR SELECTIVE PHOTOABLATION OF SURFACE". Temporal beam integrators such as a rotating dove prism or k-mirror have been used to modify the laser beam to improve the average uniformity of several laser pulses over a time interval.

U.S. Patent No. 5,646,791 to Glockler for "METHOD AND APPARATUS FOR TEMPORAL AND SPATIAL BEAM INTEGRATION", employs a spatial beam integrator for improving the spatial uniformity of a laser beam intensity profile and a separate rotating temporal beam integrator for maintaining the uniformity of the laser beam intensity over the laser pulse time interval. The spatial beam integrator includes a plurality of prisms distributed about a hollow center. The outlet face of each prism is precisely angled with respect to the body axis of the spatial beam integrator to refract the beam towards the center. The spatial beam integrator may be stationary or rotated to generate a stationary or rotated beam with respect to the spatial beam integrator. The temporal beam integrator includes a pair of rotating cylindrical lenses spaced along the beam axis by a distance substantially equal to the sum of the focal lengths of both lenses.

U.S. Patent 5,610,733 to Feldman for "BEAM-HOMOGENIZER," and U.S. Patent 4,547,037 to Case for "HOLOGRAPHIC METHOD FOR PRODUCING DESIRED WAVEFRONT TRANSFORMATION," employ diffractive optics for changing the energy distribution of laser beams. A diffractive optical element is placed in the laser beam path at a first plane. By suitably constructing a plurality of grating patterns at the first plane, a desired output energy is generated at a second plane.

DE-C-19619481, upon which the two part form of claim 1 is based, discloses the use of multiple cylindrical lens components to improve the uniformity of an excimer laser beam, and US-A-5 376 086 shows the use of diffractive elements to control laser power transmission, but not uniformity.

### SUMMARY OF THE INVENTION

The prior methods of ablating tissue employ complicated and expensive apparatus to improve uniformity of the laser beam. There is a need for a simple and inexpensive apparatus capable of transforming a beam of nonuniform intensity emitted from a pulsed laser to a laser beam with substantially uniform intensity over a large portion of the cross-section of the beam.

The present invention is set forth in claim 1, and an embodiment which is described hereinafter in detail comprises an excimer laser system for tissue ablation in which an argon fluoride excimer laser generates a rectangular nonuniform beam of pulsed laser energy along a path. The nonuniform beam has a nonuniform spatial intensity distribution. A diffractive optic diffuser is spaced from the laser and includes a transparent etched pattern disposed along the path of the beam for transforming the nonuniform beam into a spatially integrated circular beam having a substantially uniform spatial intensity distribution. A positive lens is placed downstream of the diffractive optic diffuser for focusing the spatially integrated beam to a desired spatial intensity distribution at a spatial integration plane.

This invention employs a diffractive grating technique to modify the spatial intensity distribution of an excimer laser beam. Conventional diffractive gratings include a repetitive array of diffracting elements, with apertures or obstacles, that have the effect of producing periodic alterations in the phase, amplitude, or both of an emergent wave such as a laser beam. One simple arrangement is an obstacle with a series of slits evenly spaced from each other. A more common diffractive grating device is a clear glass plate with ordered or random parallel notches scratched or ruled into the surface of the flat glass plate. The notches each serve as a source of scattered light and combine to form a regular array of parallel line sources. When the grating is totally transparent with negligible amplitude modulation, the regular variations in the optical thickness across the grating yield a modulation in phase. In that case, the diffractive grating device performs as transmission phase grating. In the present invention, a diffractive grating pattern etched in a transparent medium transforms an excimer laser beam into an output beam with a substantially uniform spatial intensity distribution.

Further features of the invention are set forth in the appended claims and described in the following description given with reference to the accompanying drawings.

### BRIEF DESCRIPTION OF THE DRAWINGS

Figure 1 is a perspective view schematically illustrating a diffractive optic apparatus in accordance with an embodiment of the present invention.
Figure 2 is a front elevational view schematically illustrating the diffractive optic apparatus of Figure 1.
Figure 3 is a perspective view schematically illustrating an embodiment of a laser beam optical delivery system incorporating the diffractive optic apparatus of Figure 1.
Figure 4 is a perspective view schematically illustrating another embodiment of a laser beam optical delivery system incorporating the diffractive optic apparatus of Figure 1.
Figure 5 is a block diagram of an ophthalmological surgery system for incorporating the invention.
Figure 6 is a plan view illustrating a scanning embodiment of the invention.
Figure 7 is a perspective view illustrating another embodiment of a beam profile having round-top spatial intensity distribution generated by the diffractive optic apparatus.
Figure 8 is a plan view of an embodiment with a lens ground on one surface of a diffractive element.

### DESCRIPTION OF THE PREFERRED EMBODIMENT

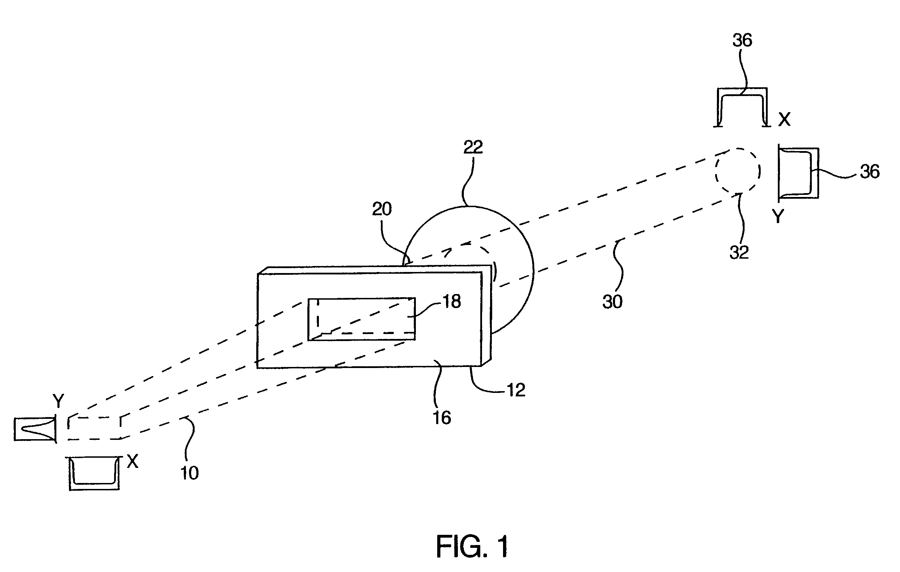

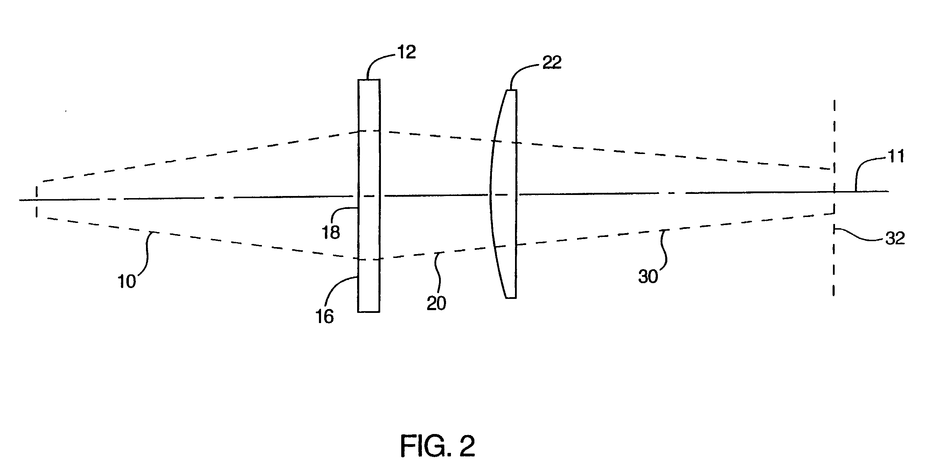

Referring to Figures 1 and 2, a generally rectangular excimer laser beam 10 is projected along the beam axis 11 toward a diffractive element 12. The intensity along the long axis (x-axis) of the beam 10 is generally uniform, while the intensity along the short axis (y-axis) is substantially gaussian. The diffractive element 12 has a generally planar body 16 that includes a transparent portion 18 which receives and diffractively transforms the laser beam 10. The diffracted beam 20 emerging from the diffractive element 12 travels along the beam axis 11 through a positive or converging lens 22 which converges the diffracted beam 20. The converged beam 30 travels along the beam axis 11 and has a transformed pattern at a spatial integration plane 32.

### Diffractive Optic Apparatus

Referring to Figure 1, the transparent portion 18 has a generally rectangular shape sized for receiving the entire rectangular beam 10. The transparent portion 18 of the diffractive element 12 has a diffractive pattern etched in a transparent medium. The transparent medium may be a glass-like silica material. The transparent medium desirably is substantially non-absorbent and non-reflective to the excimer laser beam 10. For instance, the transparent medium may include fused silica, quartz, magnesium fluoride, calcium fluoride, lithium fluoride, or sapphire.

The diffractive pattern on the transparent medium forms a diffractive grating that is configured to transform the nonuniform excimer laser beam 10 to a spatially integrated excimer beam 20 with a spatial intensity distribution that is substantially uniform across the cross-section of the beam. The cross-sectional shape of the converged beam 30 is circular. For ophthalmological surgery such as corneal ablation, the spatial intensity distribution advantageously has a top-hat shape with a circular central region that is substantially uniform and covers a large portion of the cross-section of the converged beam 30 (see the illustrated spatial intensity distribution at the spatial integration plane 32 of Figure 1). Other spatial intensity distributions are possible using different diffractive gratings.

The configuration of the diffractive pattern depends largely on the shape and spatial intensity distribution of the desired converged beam 30, and also on the characteristics of the incoming beam 10 such as its wavelength and spatial intensity distribution. The diffractive pattern may include a plurality of properly spaced etched regions such as lines, spots, or the like. For excimer lasers with short wavelengths in the neighborhood of about 193 nanometers (nm), the spacings of the etched regions in the diffractive pattern are advantageously small and precise. Known etching techniques such as dry etching may be used to etch the diffractive pattern on the transparent portion 18.

As illustrated in Figures 1 and 2, the converging lens 22 converges or focuses the diffracted beam 20 as the converged beam 30 to the spatial integration plane 32. The cross-section of the converged beam 30 at the spatial integration plane 32 is substantially circular and has a spatial intensity distribution with a top-hat profile. The uniform central region 36 of the intensity distribution desirably covers at least about 70%, more desirably close to 85%, of the cross-section of the beam 30. The size of the cross-section of the beam 30 at the spatial integration plane 32 is advantageously sized to correspond to the largest area ablated with a single laser pulse. For instance, a dimension across the cross-section of the beam 30 at the spatial integration plane 32 may typically range from 3 to 12 mm. Figures 1 and 2 show a planar convex lens 22, but other types of converging lenses 22 may be selected based on focal length to minimize aberration. An anti-reflective coating may be applied to prevent or minimize reflection of the beam 20 from the positive lens 22.

In operation, the laser beam 10 is directed along the beam axis 11 through the transparent portion 18 of the diffractive element 12 which is aligned with the laser beam 10 to receive the entire laser beam 10. The etched diffractive pattern of the transparent portion 18 serves as a diffractive control angle diffuser for altering the spatial intensity distribution of the laser beam 10. The transparent portion 18 transforms the generally rectangular gaussian laser beam 10 to the generally circular beam 20 with a substantially uniform intensity distribution. The positive lens 22 is aligned with the beam axis 11 and converges the spatially integrated beam 20 to a desired size. The cross-section of the converged beam 30 at the spatial integration plane 32 is substantially circular and uniform in spatial intensity, which is desirable for surgical procedures such as corneal ablation.

The diffractive element 12 and converging lens 22 spatially integrate the rectangular beam 10 to form the beam 30 having a substantially uniform intensity profile at the spatial integration plane. The cross-section of the beam 30 is circular, or may have other shapes. For corneal ablation, the beam 30 desirably has the uniform intensity central region 36 that covers at least about 85% of the area of the cross-section of the beam 30. The uniform intensity central region 36 includes a significant portion of the total energy of the rectangular beam 10 because there is no significant loss of energy through the diffractive optic apparatus. This renders the apparatus highly efficient.

An embodiment has been experimentally tested with satisfactory results using a 193 nm excimer laser. A binary diffractive optic 12 positioned approximately 15 mm from the converging lens 22 of 250 mm focal length produced a uniform circular beam of approximately 12 mm at the spatial integration plane 32. The binary optic employed was designed by Digital Optics Corporation of Charlotte, North Carolina. Other companies skilled in the art of diffractive optic design can produce similar gratings. The size of the spatially integrated beam at the spatial integration plane may be varied by varying the focal length of the lens 22.

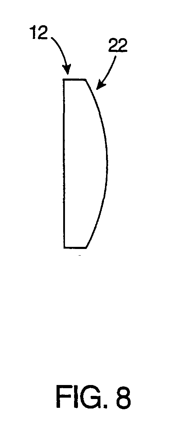

Alternate embodiments of the diffractive element 12 may be employed which do not require the use of a separate lens 22. Converging lens 22 may be ground on one surface of diffractive element 12, such as shown in Figure 8.

In an exemplary embodiment, diffractive element 12 may be rotated between pulses to provide temporal integration of the beam.

### Application in Ophthalmological Laser Surgery

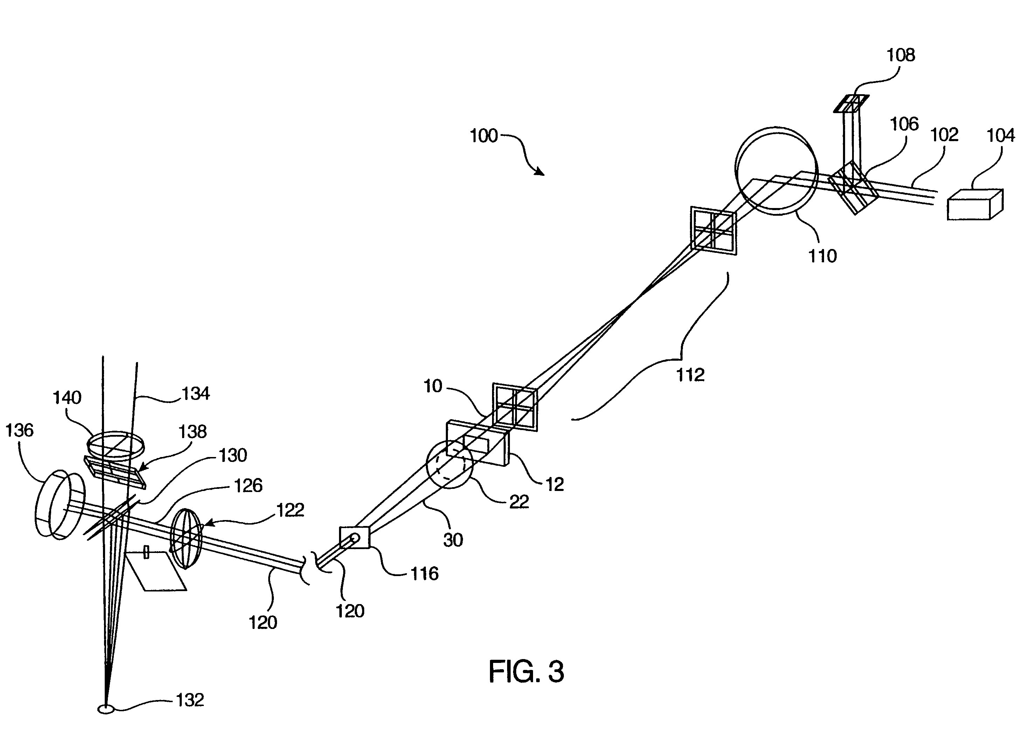

Figure 3 illustrates the application of the invention to an ophthalmological laser surgery optical system 100 and the relative orientation of the components in the system 100. The particular components and configurations described below are merely for illustrative purposes. As discussed above, the diffractive optic apparatus can be used with a variety of different excimer laser systems.

As seen in Figure 3, a beam 102 is generated from a suitable laser source 104, such as an argon fluoride (ArF) excimer laser beam source for generating a laser beam in the far ultraviolet range with a wavelength of about 193 nm. The wavelength typically ranges from about 192.5 to about 194 nm. The laser beam 102 is directed to a beam splitter 106. A portion of the beam 102 is reflected onto an energy detector 108, while the remaining portion is transmitted through the beam splitter 106 and reflected by a mirror 110 onto a rotating temporal beam integrator 112. Another type of temporal beam integrator may be used. The rotated beam emerging from the temporal integrator 112 is directed to the diffractive optic apparatus. In a preferred embodiment, the diffractive element 12 is rotated with the beam 102. In an exemplary embodiment, the diffractive element 12 is rotated at substantially the same rate as the beam 102. The beam passes through the diffractive element 12 and positive lens 22 and emerges as the converged beam 30. The converged beam 30 travels to the spatial integration plane 32 at which a variable aperture 116 is disposed. The spatial integration plane 32 is disposed near the focal point of the positive lens 22. A beam 120 emerges from the variable aperture 116. The variable aperture 116 is desirably a variable diameter iris combined with a variable width slit (not shown) used to tailor the size and profile of the beam 30 to a particular ophthalmological surgery procedure, such as photorefractive keratectomy (PRK) and phototherapeutic keratectomy (PTK).

The beam 120 is directed onto an imaging lens 122, which may be a biconvex singlet lens with a focal length of about 125 mm. The imaged beam 126 emerging from the imaging lens 122 is reflected by a mirror/beam splitter 130 onto the surgical plane 132. The apex of the cornea of the patient is typically positioned at the surgical plane 132. Imaging lens 122 may be moved transverse to the beam to offset the imaged beam in order to scan the imaged beam about the surgical plane 132. A treatment energy detector 136 senses the transmitted portion of the beam energy at the mirror/beam splitter 130. A beam splitter 138 and a microscope objective lens 140 form part of the observation optics. If desired, a beam splitter may be installed in the optical path of the beam 134 emanating from the microscope objective lens. The beam splitter is optically coupled to a video camera to assist in viewing or recording the surgical procedure. Similarly, a heads-up display may also be inserted in the optical path of the microscope objective lens 140 to provide an additional observational capability. Other ancillary components of the laser optical system 100 which are not necessary to an understanding of the invention such as the movable mechanical components driven by an astigmatism motor and an astigmatism angle motor, have been omitted to avoid prolixity.

The diffractive optic apparatus which comprises the diffractive element 12 and positive lens 22 may be used for different laser systems, including scanning laser and large area laser ablation systems. An example is. the VISX STAR Excimer Laser System", which is commercially available from VISX, Incorporated of Santa Clara, California. This system produces an output of 193.0 nm, operates at a frequency of 6.0 Hz, and is adjusted to deliver uniform fluence of 160.0 millijoules/cm² with a 6.0 mm diameter ablation zone. Other laser systems include the T-PRK^{R} scanning and tracking laser from Autonomous Technologies Corporation, the SVS Apex laser from Summit Technology Inc., the Keracor" 117 scanning laser system from Chiron Vision, and the like.

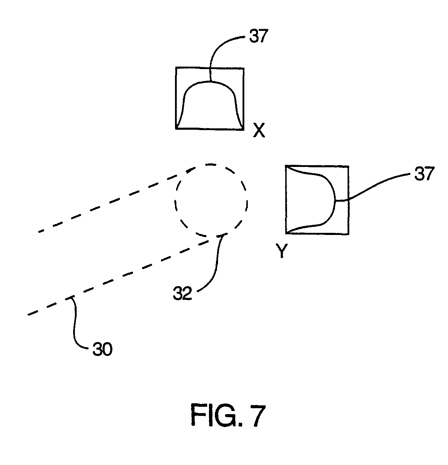

In an alternate embodiment, the converged beam 30 may produce a central region with a round-top spatial intensity distribution 37 at the spatial integration plane 32, as shown in Figure 7. This round-top distribution 37 may be created by varying the separation among the converging lens 22, diffractive element 12, and spatial integration plane 32. Alternatively, a different diffractive pattern on the diffractive element 12 may be employed.

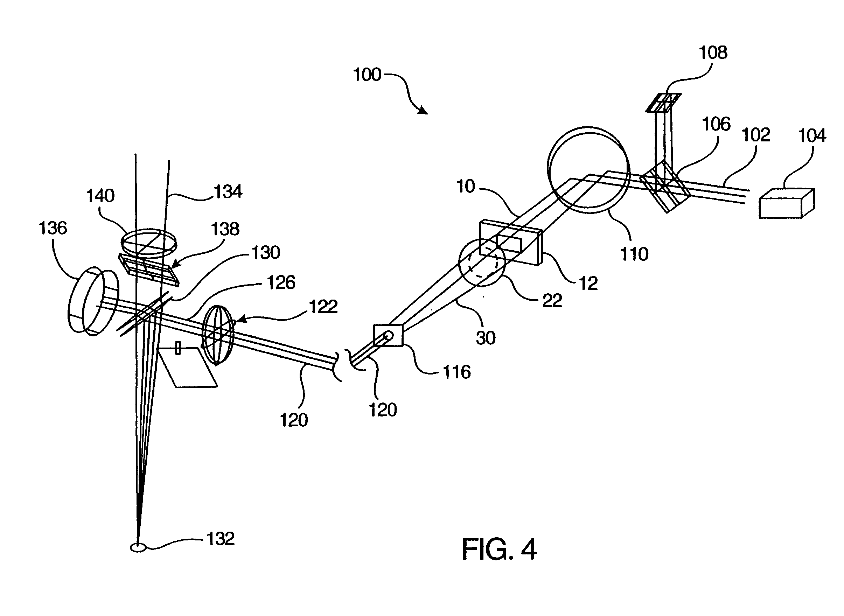

The spatially integrated beam 30 may desirably be exceptionally uniform over nearly 85% of the area of the cross-section of the beam 30 during the laser pulse time interval of the beam 30. For such a spatially integrated beam 30, the temporal beam integrator 112 may be eliminated without adverse effects on the characteristics of the beam 30 and operation of the laser system 100. In that case, the diffractive optic apparatus comprising the diffractive element 12 and positive lens 22 serves as the spatial beam integrator and does not require the temporal beam integrator. Figure 4 illustrates an embodiment of the laser optic system 100 without the rotating temporal beam integrator 112 of Figure 3.

The diffractive optic apparatus is simple and inexpensive, and does not require rotation by a machine such as a motor. The diffractive element 12 and positive lens 22 can be easily aligned with the beam axis 11. The simple diffractive optic apparatus is easy to use and maintain. In an exemplary embodiment, however, the diffractive optic apparatus may be rotated to provide both spatial and temporal beam integration. The diffractive optic apparatus may be adapted for different excimer laser systems.

The ophthalmological laser surgery optical system 100 may employ the ultraviolet laser beam in corneal ablation procedures to ablate corneal tissue in a photodecomposition that does not cause thermal damage to adjacent and underlying tissue. Molecules at the irradiated surface are broken into smaller volatile fragments without heating the remaining substrate; the mechanism of the ablation is photochemical, i.e. the direct breaking of intermolecular bonds. The ablation removes a layer of the stroma to change its contour for various purposes, such as correcting myopia, hyperopia, and astigmatism. Such systems and methods are disclosed in the following U.S. patents and patent applications: U.S. Pat. No. 4,665,913 issued May 19, 1987 for "METHOD FOR OPHTHALMOLOGICAL SURGERY"; U.S. Pat. No. 4,669,466 issued June 2, 1987 for "METHOD AND APPARATUS FOR ANALYSIS AND CORRECTION OF ABNORMAL REFRACTIVE ERRORS OF THE EYE"; U.S. Pat. No. 4,732,148 issued March 22, 1988 for "METHOD FOR PERFORMING OPHTHALMIC LASER SURGERY"; U.S. Pat. No. 4,770,172 issued September 13, 1988 for "METHOD OF LASER-SCULPTURE OF THE OPTICALLY USED PORTION OF THE CORNEA"; U.S. Pat. No. 4,773,414 issued September 27, 1988 for "METHOD OF LASER-SCULPTURE OF THE OPTICALLY USED PORTION OF THE CORNEA"; U.S. Patent Application Serial No. 109,812 filed October 16, 1987 for "LASER SURGERY METHOD AND APPARATUS"; U.S. Patent No. 5,163,934 issued November 17, 1992 for "PHOTOREFRACTIVE KERATECTOMY"; U.S. Patent No. 5,556,395 issued September 17, 1996 for "METHOD AND SYSTEM FOR LASER TREATMENT OF REFRACTIVE ERROR USING AN OFFSET IMAGE OF A ROTATABLE MASK"; U.S. Patent Application Serial No. 08/368,799, filed January 4, 1995 for "METHOD AND APPARATUS FOR TEMPORAL AND SPATIAL BEAM INTEGRATION"; U.S. Patent Application Serial No. 08/138, 552, filed October 15, 1993 for "METHOD AND APPARATUS FOR COMBINED CYLINDRICAL AND SPHERICAL EYE CORRECTIONS"; and U.S. Patent Application Serial No. 08/058,599, filed May 7, 1993 for "METHOD AND SYSTEM FOR LASER TREATMENT OF REFRACTIVE ERRORS USING OFFSET IMAGING".

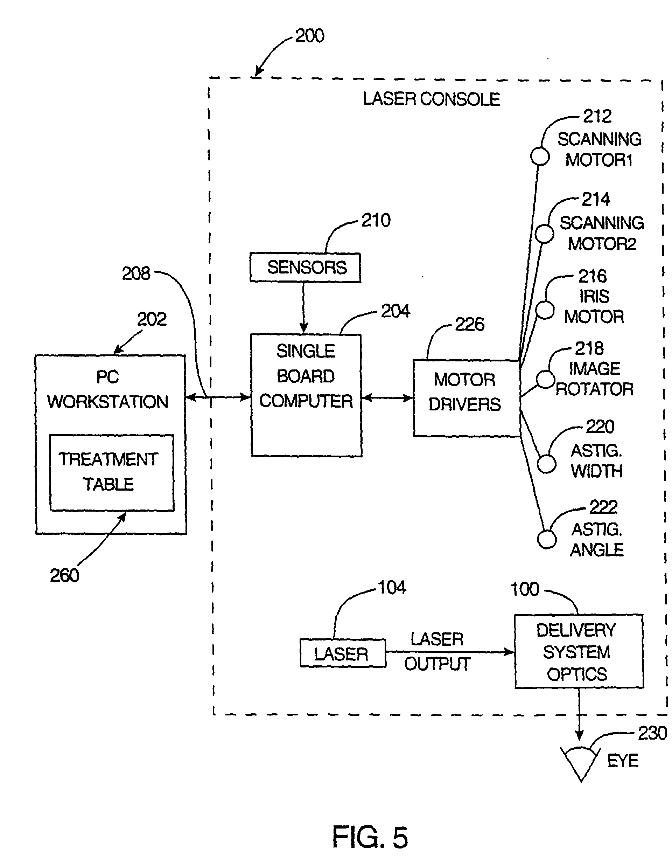

The block diagram of Figure 5 illustrates an ophthalmological surgery system 200 for incorporating the invention that includes a personal computer (PC) work station 202 coupled to a single board computer 204 of the laser surgery system 200 by means of a first bus connection 208. The PC work station 202 and the subcomponents of the laser surgery unit 200 are known components and may comprise the elements of the VISX TWENTY/TWENTY EXCIMER LASER SYSTEM or the VISX STAR Excimer Laser System", which are available from Visx, Incorporated of Santa Clara, California. The laser surgery system 200 includes a plurality of sensors generally designated with reference numeral 210 which produce feedback signals from the movable mechanical and optical components in the ophthalmological laser surgery optical system 100 of Figure 3 or Figure 4. The movable mechanical and optical components include, for example, the elements driven by an iris motor 216, an image rotator 218, and astigmatism width motor 220, and an astigmatism angle motor 222. For scanning treatments where an ablation from an individual laser pulse is variably offset from the treatment center, scanning motor 1 (212) and scanning motor 2 (214) are provided. The moving lens 122 transverse to the beam 120 will provide this variable offset. The feedback signals from the sensors 210 are provided via appropriate signal conductors to the single board computer 204, which is desirably an STD bus compatible single board computer using a type 8031 microprocessor. The single board computer 204 controls the operation of the motor drivers generally designated with reference numerals 226 for operating the elements 216, 218, 220, and 222. In addition, the single board computer 204 controls the operation of the excimer laser 104, which is desirably an ArF laser with a 193 nanometer wavelength output designed to provide feedback stabilized fluence of 160 milliJoules per cm² at the cornea of the patient's eye 230 via the delivery system optics 100 of Figure 3 or Figure 4. Other ancillary components of the laser surgery system 200 which are not necessary to an understanding of the invention, such as a high resolution microscope, a video monitor for the microscope, a patient eye retention system, and an ablation effluent evacuator/filter, as well as the gas delivery system, have been omitted to avoid prolixity. Similarly, the keyboard, display, and conventional PC subsystem components, such as flexible and hard disk drives, memory boards and the like, have been omitted from the depiction of the PC work station 202.

The laser surgery system 200 may be used for procedures such as photorefractive keratectomy (PRK) and phototherapeutic keratectomy (PTK). Using PC workstation 202, an operator enters at least one patient treatment parameter such as the desired change in patient refraction. The above treatment parameter corresponds to an improved change corneal shape. The PC workstation 202 may then calculate treatment table 260 containing the positions of the laser elements during laser treatment. The laser elements typically varied during treatment include variable aperture 116 and the position of the lens 112. In PRK, for instance, the laser surgery system 200 is used to ablate the tissue of the cornea after removal of the epithelium. To correct for myopia, the circular laser beam 30 is adjusted to a circular spot registered with the treatment area on the cornea using the adjustable aperture 116. The circular spot is typically a 0.5-6 mm circle. The correction for myopia reduces the radius of curvature of the cornea. This requires removal of more tissue in the center of the cornea and less tissue toward the peripheral treatment area. A first pulse of the apertured beam 120 can ablate away tissue from the entire treatment area, but successive pulses are reduced in diameter by the variable aperture 116 so that the pulses become successively smaller. In another embodiment, successive pulses are incrementally increased from a small to large diameter covering the treatment area. This removes more tissue from the central region and brings the cornea to the desired contour having a decreased curvature. After the photorefractive keratectomy procedure, the epithelium rapidly regrows over the shaped area, producing a new anterior surface of the cornea. Alternatively, the epithelium is not removed but is partially severed and moved to the side for surgery and returned to its original position after the PRK.

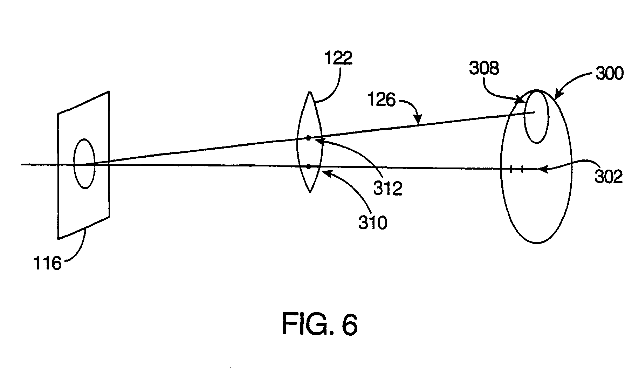

In an alternate embodiment shown in Figure 6, the treatment area 300 of the cornea comprises a plurality of smaller areas ablated with individual laser pulses, such as the offset imaged apertured beam 126. The positions and sizes of the smaller ablated areas correspond to the values calculated in the treatment table 260. The decrease in curvature is accomplished by the scanning beam 126 about the cornea. As shown in Figure 6, the offset position 312 of the lens 122 is varied about the central position 310. This scanning produces an offset imaged apertured beam 126 with an outer portion 308. Desirably, the beam 126 covers the center 302 of the treatment area 300 during a portion of the scanning treatment. Optionally, a dimension of the variable aperture 116 may be varied during scanning to vary the size of the beam 126. In a preferred embodiment, the diffractive optic 12 is moved so as to rotate between pulses. In an exemplary embodiment, the beam rotator 112 and diffractive optic 12 are rotated between pulses. The successive pulses of the scanning beam contour the desired decreased curvature according'to the treatment table 260.

For correcting hyperopia, the beam 120 of Figure 3 or Figure 4 scans over a treatment area of the cornea. As shown in Figure 6, the treatment area 300 of the cornea comprises a plurality of smaller areas ablated with individual laser pulses, such as the offset imaged apertured beam 126. The positions and sizes of the smaller ablated areas correspond to the values calculated in the treatment table 260. More tissue must be removed from the periphery of the treatment area than from the center. This increases the radius of curvature of the cornea. The increase in curvature is accomplished by scanning the beam 126 about the cornea. As shown in Figure 6, the offset position 312 of the lens 122 is varied about the central position 310. This-scanning produces an offset images apertured beam 126 with an outer portion 308. Desirably, the beam 126 does not cover the center 302 of the treatment area 300 during a portion of the scanning treatment. Optionally, a dimension of variable aperture 116 may be varied during scanning to vary the size of the beam 126. In a preferred embodiment, the diffractive optic 12 is moved so as to rotate between pulses. In an exemplary embodiment, the beam rotator 112 and diffractive optic 12 are rotated between pulses. Successive pulses of the scanning beam contour the cornea to the desired increased curvature according to the treatment table 260.

For correcting astigmatic properties of the cornea, the variable width slit (not shown) diametrically spans the treatment area of the cornea which is generally rectangular. The first pulse of the imaged apertured beam 126 ablates away a generally rectangular area of corneal tissue. Successive pulses are directed with varying width of the generally rectangular spot of the imaged apertured beam 126 which are symmetrically positioned with respect to the optical center. The astigmatism correcting change is effected by volumetric removal of the corneal tissue.

While the above provides a full and complete disclosure of the preferred embodiments of the invention, various modifications, alternate constructions and equivalents may be employed as desired. For example, while the beam passed through the variable aperture 116 is offset by transverse motion of the imaging lens 122 in the preferred embodiment, other scanning elements such as rotating mirrors and prisms may be employed if desired. Further, lasers of appropriate wavelengths other than the laser 104 may be used, if desired and effective. Therefore, the above description and illustrations should not be construed as limiting the invention, which is defined by the appended claims.

## Claims

1. An excimer laser system for tissue ablation comprising:
an excimer laser (104) for generating a generally rectangular non-uniform beam (102) of pulsed laser energy along a path, the non-uniform beam having a non-uniform spatial intensity distribution; and
means (10,12) disposed in the path of the non-uniform beam for transforming the non-uniform beam into a spatially integrated beam having a predetermined substantially uniform spatial intensity distribution;
and a positive lens (22) positioned for focusing the beam from the diffractive optic diffuser (12) onto a spatial integration plane;
**characterized in that**:
said transforming means (10,12) comprises a diffractive optic diffuser (12) spaced from the laser (104) and including a diffractive pattern disposed across the path of the beam and arranged to transform the generally rectangular non-uniform beam such that said spatially integrated beam has a substantially circular spatial intensity distribution in cross-section.

2. An excimer laser system as claimed in claim 1, wherein the laser (104) is an ArF laser having a wavelength of about 193 nm.

3. An excimer laser system as claimed in claim 1 or 2, wherein the non-uniform beam (102) has a substantially gaussian spatial intensity distribution along one axis.

4. An excimer laser system as claimed in any preceding claim, wherein the substantially circular spatial intensity distribution has a top-hat profile with a uniform central region.

5. An excimer laser system as claimed in claim 4, wherein the uniform central region covers about 70% to 85% of the cross-section of the spatially integrated beam.

6. An excimer laser system as claimed in any preceding claim, wherein the diffuser (12) includes a transparent portion (18) having said diffractive pattern formed thereon.

7. An excimer laser system as claimed in claim 6, wherein the diffractive pattern comprises a plurality of spaced etched lines.

8. An excimer laser system as claimed in claim 6 or 7, wherein the transparent portion (18) is generally rectangular.

9. An excimer laser system as claimed in claim 6 or 7 or 8, wherein the transparent portion (18) includes a transparent material comprising quartz, fused silica, magnesium fluoride, calcium fluoride, lithium fluoride or sapphire.

10. An excimer laser system as claimed in any preceding claim wherein the positive lens (22) is ground on one surface of the diffractive optic diffuser (12).

11. An excimer laser system as claimed in any preceding claim wherein the spatial integration plane is disposed about the focal point of the positive lens (22).

12. An excimer laser system as claimed in any preceding claim for reshaping the cornea of an eye, the system comprising:
an aperture (116) spaced from the diffractive optic diffuser (12) generally at the spatial integration plane for passing an area of the spatially integrated beam; and
a positive lens (122) spaced from the aperture (116) to form an image of the passed beam at a second plane (132).

13. An excimer laser system as claimed in claim 12, comprising means (216) for adjusting a dimension of the aperture (116) between laser pulses.

14. An excimer laser system as claimed in claim 12 or 13, further comprising scanning means (212,214) for displacing the image of the passed beam.

15. An excimer laser system as claimed in claim 14, further comprising control means (204) for the scanning means (212,214) arranged to displace the image according to a treatment table (260).

16. An excimer laser system as claimed in any of the preceding claims, further comprising means (218) for moving the diffractive optic diffuser (12) between laser pulses.

17. An excimer laser system as claimed in claim 16, wherein the moving means (218) is arranged to effect rotation of the diffractive optic diffuser (12).

## Patentansprüche

1. Excimerlasersystem zur Ablation von Gewebe, mit:
einem Excimer-Laser (104) zum Erzeugen eines in Allgemeinen rechteckigen nicht gleichförmigen Strahls (102) gepulster Laserenergie entlang eines Weges, wobei der nicht gleichförmige Strahl eine nicht gleichmäßige räumliche Intensitätsverteilung aufweist, und
einer Einrichtung (10, 12), die in dem Weg des nicht gleichförmigen Strahls angeordnet ist, um den nicht gleichförmigen Strahl in einen räumlich integrierten Strahl umzuwandeln, der eine vorbestimmte im Wesentlichen gleichmäßige räumliche Intensitätsverteilung aufweist, und
einer Sammellinse (22), die angeordnet ist, um den Strahl von dem streuoptischen Diffusor (12) auf eine räumliche Integrationsebene zu fokussieren,
**gekennzeichnet dadurch, dass**:
die Umwandlungseinrichtung (10, 12) einen streuoptischen Diffusor (12) umfasst, der von dem Laser (104) beabstandet ist und ein Streumuster aufweist, das quer zu dem Weg des Strahls angeordnet und ausgelegt ist, um den im Allgemeinen rechteckigen nicht gleichförmigen Strahl so umzuwandeln, dass der räumlich integrierte Strahl eine im Wesentlichen kreisförmige räumliche Intensitätsverteilung im Querschnitt aufweist.

2. Excimerlasersystem nach Anspruch 1, bei dem der Laser (104) ein ArF-Laser mit einer Wellenlänge von etwa 193 nm ist.

3. Excimerlasersystem nach Anspruch 1 oder 2, bei dem der nicht gleichförmige Strahl (102) entlang einer Achse eine im Wesentlichen Gaußsche räumliche Intensitätsverteilung aufweist.

4. Excimerlasersystem nach einem der vorherigen Ansprüche, bei dem die im Wesentlichen kreisförmige räumliche Intensitätsverteilung ein Hutprofil mit einem gleichförmigen zentralen Bereich aufweist.

5. Excimerlasersystem nach Anspruch 4, bei dem der gleichförmige zentrale Bereich etwa 70 % bis 85 % des Querschnitts des räumlich integrierten Strahls abdeckt.

6. Excimerlasersystem nach einem der vorherigen Ansprüche, bei dem der Diffusor (12) einen transparenten Bereich (18) umfasst, der das Streumuster darauf ausgebildet aufweist.

7. Excimerlasersystem nach Anspruch 6, bei dem das Streumuster eine Mehrzahl von beabstandeten geätzten Linien umfasst.

8. Excimerlasersystem nach Anspruch 6 oder 7, bei dem der transparente Bereich (18) im Allgemeinen rechteckig ist.

9. Excimerlasersystem nach Anspruch 6 oder 7 oder 8, bei dem der transparente Bereich (18) ein transparentes Material mit Quarz, Quarzglas, Magnesiumfluorid, Kalziumfluorid, Lithiumfluorid oder Saphir aufweist.

10. Excimerlasersystem nach einem der vorherigen Ansprüche, bei dem die Sammellinse (22) auf einer Oberfläche des streuoptischen Diffusors (12) geschliffen ist.

11. Excimerlasersystem nach einem der vorherigen Ansprüche, bei dem die räumliche Integrationsebene etwa an dem Fokuspunkt der Sammellinse (22) angeordnet ist.

12. Excimerlasersystem nach einem der vorherigen Ansprüche zum Umformen der Kornea eines Auges, wobei das System umfasst:
eine Öffnung (116), die von dem streuoptischen Diffusor (12) im Allgemeinen an der räumlichen Integrationsebene beabstandet ist, um einen Bereich des räumlich integrierten Strahls durchzulassen, und
eine Sammellinse (122), die von der Öffnung (116) beabstandet ist, um ein Bild des durchgelassenen Strahls auf einer zweiten Ebene (132) zu bilden.

13. Excimerlasersystem nach Anspruch 12, mit einer Einrichtung (216) zum Einstellen einer Abmessung der Öffnung (116) zwischen Laserpulsen.

14. Excimerlasersystem nach Anspruch 12 oder 13, ferner mit einer Abtasteinrichtung (212, 214), um das Bild des durchgelassenen Strahls zu versetzen.

15. Excimerlasersystem nach Anspruch 14, ferner mit einer Steuereinrichtung (204) für die Abtasteinrichtung (212, 214), die ausgelegt ist, das Bild gemäß einer Behandlungstabelle (260) zu versetzen.

16. Excimerlasersystem nach einem der vorherigen Ansprüche, ferner mit einer Einrichtung (218) zum Bewegen des streuoptischen Diffusors (12) zwischen Laserpulsen.

17. Excimerlasersystem nach Anspruch 16, bei dem die Bewegungseinrichtung (218) ausgelegt ist, eine Drehung des streuoptischen Diffusors (12) zu bewirken.

## Revendications

1. Système de laser à excimère pour l'ablation de tissus, comprenant :
un laser à excimère (104) pour générer un faisceau non uniforme généralement rectangulaire (102) d'énergie laser pulsée le long d'un trajet, le faisceau non uniforme ayant une distribution d'intensité spatiale non uniforme ; et
un moyen (10, 12) disposé sur le trajet du faisceau non uniforme pour transformer le faisceau non uniforme en un faisceau intégré dans l'espace ayant une distribution d'intensité spatiale sensiblement uniforme prédéterminée ; et
une lentille positive (22) positionnée pour focaliser le faisceau issu du diffuseur optique diffractif (12) sur un plan d'intégration spatiale ;
**caractérisé en ce que** :
ledit moyen de transformation (10, 12) comprend un diffuseur optique diffractif (12) espacé du laser (104) et comprenant un motif diffractif disposé sur le trajet du faisceau et arrangé pour transformer le faisceau non uniforme généralement rectangulaire de manière à ce que ledit faisceau intégré dans l'espace ait une distribution d'intensité spatiale dont la section transversale est sensiblement circulaire.

2. Système de laser à excimère, tel que revendiqué dans la revendication 1, dans lequel le'laser (104) est un laser ArF ayant une longueur d'onde d'environ 193 nm.

3. Système de laser à excimère, tel que revendiqué dans les revendications 1 ou 2, dans lequel le faisceau non uniforme (102) a une distribution d'intensité spatiale sensiblement gaussienne le long d'un axe.

4. Système de laser à excimère, tel que revendiqué dans l'une quelconque des revendications précédentes, dans lequel la distribution d'intensité spatiale sensiblement circulaire a un profil en cloche avec une région centrale uniforme.

5. Système de laser à excimère, tel que revendiqué dans la revendication 4, dans lequel la région centrale uniforme couvre environ 70 % à 85 % de la section transversale du faisceau intégré dans l'espace.

6. Système de laser à excimère, tel que revendiqué dans l'une quelconque des revendications précédentes, dans lequel le diffuseur (12) comprend une partie transparente (18) sur laquelle est formé ledit motif diffractif.

7. Système de laser à excimère, tel que revendiqué dans la revendication 6, dans lequel le motif diffractif comprend une pluralité de lignes gravées espacées.

8. Système de laser à excimère, tel que revendiqué dans les revendications 6 ou 7, dans lequel la partie transparente (18) est généralement rectangulaire.

9. Système de laser à excimère, tel que revendiqué dans les revendications 6, 7 ou 8, dans lequel la partie transparente (18) comprend un matériau transparent comprenant du quartz, du verre de silice, du fluorure de magnésium, du fluorure de calcium, du fluorure de lithium ou du saphir.

10. Système de laser à excimère, tel que revendiqué dans l'une quelconque des revendications précédentes, dans lequel la lentille positive (22) est polie sur une surface du diffuseur optique diffractif (12).

11. Système de laser à excimère, tel que revendiqué dans l'une quelconque des revendications précédentes, dans lequel le plan d'intégration spatiale est disposé près du point focal de la lentille positive (22).

12. Système de laser à excimère, tel que revendiqué dans l'une quelconque des revendications précédentes, et destiné à remettre en forme la cornée d'un oeil, le système comprenant :
une ouverture (116) espacée du diffuseur optique diffractif (12) généralement dans le plan d'intégration spatiale pour laisser passer une surface du faisceau intégré dans l'espace ; et
une lentille positive (122) espacée de l'ouverture (116) pour former, au niveau d'un second plan (132), une image du faisceau transmis.

13. Système de laser à excimère, tel que revendiqué dans la revendication 12, comprenant un moyen (216) pour régler une dimension de l'ouverture (116) entre les impulsions laser.

14. Système de laser à excimère, tel que revendiqué dans les revendications 12 ou 13, comprenant en outre un moyen d'analyse (212, 214) pour déplacer l'image du faisceau transmis.

15. Système de laser à excimère, tel que revendiqué dans la revendication 14, comprenant en outre un moyen de commande (204) pour le moyen d'analyse (212, 214) arrangé pour déplacer l'image selon une table de traitement (260).

16. Système de laser à excimère, tel que revendiqué dans l'une quelconque des revendications précédentes, comprenant en outre un moyen (218) pour déplacer le diffuseur optique diffractif (12) entre les impulsions laser.

17. Système de laser à excimère, tel que revendiqué dans la revendication 16, dans lequel le moyen de déplacement (218) est arrangé pour appliquer une rotation au diffuseur optique diffractif (12).
